# EUROPEAN PATENT SPECIFICATION

(11) **EP 1 962 758 B1**
(45) Date of publication and mention of the grant of the patent: **10.02.2010**
(21) Application number: 06842618.8
(22) Date of filing: 19.12.2006
(51) Int. Cl.: A61F 13/42, A61F 13/84, A61F 13/15

(54) **DISPOSABLE ABSORBENT ARTICLES HAVING A PARTIALLY VISIBLE GRAPHIC**
SAUGFÄHIGER EINWEGARTIKEL MIT EINER TEILWEISE SICHTBAREN GRAFIK
ARTICLES ABSORBANTS JETABLES DOTES D'UN GRAPHIQUE PARTIELLEMENT VISIBLE

(30) Priority: 20.12.2005 US 752072 P
(43) Date of publication of application: 03.09.2008
(73) Proprietor: The Procter and Gamble Company, Cincinnati, Ohio 45202 (US)
(72) Inventor: LIU, Kuangkai, Cincinnati, Ohio 45249 (US)
(74) Representative: Heide, Ute
(86) International application number: PCT/IB2006/054960
(87) International publication number: WO 2007/072431

(56) References cited:
- EP-A- 0 148 115
- EP-A- 1 547 560
- EP-A- 1 552 802
- EP-A- 1 591 091
- EP-A1- 1 216 673
- WO-A-2006/073522
- WO-A-2006/073523

## Description

### FIELD OF THE INVENTION

The present invention relates to a disposable absorbent article comprising a backsheet having a garment facing surface and a bodyfacing surface, a topsheet having a gannet facing surface and a bodyfacing surface, an absorbent core having two opposing longitudinal edges and two opposing transverse edges and wherein said core is disposed between said bodyfacing surface of said backsheet and said garment facing surface of said topsheet; and wherein a graphic is disposed on said bodyfacing surface of said backsheet and is partially covered by said core.

### BACKGROUND OF THE INVENTION

More often than not disposable absorbent articles are incorporating graphics as an ordinary feature. In most instances, these graphics are applied to the backsheet of the product so that the graphics are visible while the product is being worn. The graphics have typically been printed directly on a component of the backsheet or have been printed on a separate layer, such as a tissue layer, which is disposed on the backsheet.

Processes for attaching indicator elements or inner layer webs to the outer sheet of a diaper are described in EP 1 552 802 and EP 1 547 560.

EP 1 591 091 describes disposable diapers having patterned sheets.

EP 1 215 673 relates to a gate system for use in diapers and other absorbent garments.

WO 2006/073523 discloses disposable absorbent articles with interactive graphics.

There are many reasons to incorporate graphics in disposable absorbent articles. For instance, the graphics can improve the appearance and appeal of the product, to both the wearer and the purchaser. Graphics can also impact the manner in which a disposable absorbent article is used. For example, graphics on disposable diapers can be used by caregivers to amuse and/or educate a child during diapering. Similarly, graphics on disposable training pants can provide educational and motivational mechanisms to facilitate the toilet training process. Graphics on training pants can also serve to increase the child's interest in the product and thereby increase the child's interest in the toilet training process.

Graphics can further be used to indicate when a urine insult has occurred. For instance, it has been known in the art that upon wetting, disposable absorbent articles like diapers have included graphics that appear or disappear to indicate the insult. To date, however, it has not been possible to deliver disposable absorbent products which include printed graphics that can be disposed directly onto a film portion of the backsheet of an absorbent product and have the graphic ultimately appear clearly to the unaided eye of a consumer and wearer when exposed to liquid. Additionally, it is envisioned that such an article would entice a wearer to participate in the donning process of the article since a game of "peek-a-boo" is mimicked by the partially hidden graphic.

### SUMMARY OF THE INVENTION

The present invention, therefore, relates to disposable absorbent articles comprising:
a) a backsheet having a garment facing surface and a bodyfacing surface;
b) a topsheet having a garment facing surface and a bodyfacing surface;
c) an absorbent core having two opposing longitudinal edges and two opposing transverse edges and wherein said core is disposed between said bodyfacing surface of said backsheet and said garment facing surface of said topsheet; and wherein a graphic is disposed on said bodyfacing surface of said backsheet and is partially covered by said core.

### BRIEF DESCRIPTION OF THE DRAWINGS

Figure 1 shows a top plan view of an absorbent article of the present invention with the bodyfacing surface of the topsheet visible.
Figure 2 shows a top plan view of the absorbent article of Figure 1 with the garment facing surface of the backsheet visible.

### DETAILED DESCRIPTION OF THE INVENTION

### DEFINITIONS

As used herein, the term "absorbent articles" refers to devices that absorb and contain body exudates, and, more specifically, refers to devices that are placed against or in proximity to the body of the wearer to absorb and contain the various exudates discharged from the body. Absorbent articles may include diapers, training pants, adult incontinence undergarments, feminine hygiene products, breast pads, care mats, bibs, wound dressing products, and the like. As used herein, the term "body fluids" or"body exudates" includes, but is not limited to, urine, blood, vaginal discharges, breast milk, sweat and fecal matter.

As used herein, the term "absorbent core" refers to the component of the absorbent article that is primarily responsible for fluid handling properties of the article, including acquiring, transporting, distributing and storing body fluids. As such, the absorbent core typically does not include the topsheet, backsheet or outer cover of the absorbent article.

As used herein, the term "bonded" refers to different materials being attached (cohesively or adhesively) in at least a portion thereof. The attached portions may be random or may have a pattern such as stripes, spirals, dots, and the like. The attached portions may be located at the peripheries, throughout the surface area, or both. Suitable attachment means known in the art may be used, including but not limited to adhesives, heat, pressure, crimping, ultrasonic, chemical (via hydrogen bonds or other cohesive forces), mechanical (e.g., fasteners, entanglements), hydraulic, vacuum and combinations thereof.

As used herein, the term "composite structure" refers to a multi-region structure wherein the materials comprising the regions may be operatively associated or bonded. The regions may even be in intimate contact such that the composite has a unitary structure. Further, the regions may be positioned in a layered (face-to-face) arrangement, or a side-by-side arrangement.

As used herein, the term "disposable" is used in its ordinary sense to mean an article that is disposed or discarded after a limited number of usage events over varying lengths of time, such as less than about 20 events, less than about 10 events, less than about 5 events, less than about 2 events, or even about 1 event.

As used herein, the term "disposed' and variations thereof are intended to mean that one element can be integral with another element, or that one element can be a separate structure bonded to or placed with or placed near another element.

As used herein, the term "intimate bonding" refers to physical contact between two layers such that they resist separation with no readily visually identifiable areas of separation. In one particular embodiment, the inner and outer layers are adhesively laminated together in the graphic regions using a meltblowing process to form an overlapping network of adhesive filaments.

As used herein, the term 'joined' encompasses configurations wherein an element is directly secured to the other element by affixing the element directly to the other element, and configurations wherein the element is indirectly secured to the other element by affixing the element to intermediate member(s), which in turn are affixed to the other element.

The term "macroporous" refers to materials having pores too large to effect capillary transport of fluid, generally having pores greater than about 0.5 mm in diameter and, more specifically, having pores greater than about 1.0 nun in diameter.

As used herein, the terms "meltblown" and "meltblown web" refer to a web having fibers formed by extruding a molten thermoplastic material through a plurality of fine, usually circular, die capillaries as molten threads or filaments into converging high velocity heated gas/air streams which attenuate the molten filaments to reduce their diameter. The reduction in fiber diameter is substantially greater then the reduction of fiber diameter in the spunhonding process, resulting in microfibers having average fiber diameter larger than 0.2 microns and typically in the range of 0.6 to 10 microns. Thereafter, the meltblown fibers are carried by the high velocity gas stream and are deposited on a collecting surface to form a web of randomly disbursed fibers. Various meltblown processes are known in the art.

As used herein, the term "microporous" refers to materials which are capable of transporting fluids by capillary action.

As used herein, the terms "nonwoven" and "nonwoven web" refers to a web that has a structure of individual fibers which are interlaid forming a matrix, but not in an identifiable repeating manner. Nonwoven webs may be formed by a variety of processes known to those skilled in the art, for example, meltblowing, spunbonding, wet-laying, air-laying, and various bonding-carding processes.

As used herein, the term "operatively associated' refers to a structure comprising different materials positioned at least in partial contact with each other in use. The materials are physically separable and each exhibits properties that can be measured individually. The materials may be arranged in a face-to-face relationship in the z-dimension, or in a side-by-side relationship in the xy-dimension.

As used herein, the term "pulp" or "cellulosic fibers" include those natural fiber derived from trees or vegetations (e.g., hardwood fibers, softwood fibers, hemp, cotton, flax, esparto grass, milkweed, straw, bagasse and the like), their processed/regenerated fibers (e.g., Rayon®) or chemically derivatized fibers (e.g., cellulose esters), and combinations thereof. Suitable hardwood fibers include eucalyptus fibers. Suitable hardwood fibers may be prepared by kraft or other chemical pulping methods. Suitable softwood fibers include southern softwood (SS) fibers and northern softwood (NS) fibers. Softwood fibers for use herein can be chemically (e.g., without limitation, kraft pulp) or mechanically pulped (e.g., without limitation, chemithermial mechanical pulp (CTMP) and thermal mechanical pulp (TMP)).

As used herein, the term "region" refers to a zone or an area comprising a material being physically, chemically, or visually distinguishable from surrounding or adjoining materials. Various regions of materials may include transitional regions in between. The regions may be positioned in the z-dimension or in the xy-dimension. As used herein, the term "z-dimension" refers to the dimension orthogonal to the length and width of the structure or article. The z-dimension usually corresponds to the thickness of the structure or article. As used herein, the term "xy-dimension" refers to the plane orthogonal to the thickness of the member, core or article when the member, core or article is in a flat-out state. The xy-dimension usually corresponds to the length and width, respectively, of the structure or article in a flat-out state.

As used herein, the terms "spunbond' and "spunbonded web" refers to a web having fibers formed by extruding a molten thermoplastic material as filaments from a plurality of fine capillaries of a spinnerette having a circular or other configuration, then rapidly reducing the diameter of the extruded filaments by fluid drawing or other well known spunbonding mechanisms. Spunbond fibers are quenched and generally not tacky when they are deposited onto a collecting surface. Spunbond fibers are generally continuous and often have average between about 10 to about 30 microns.

As used herein, the term "unitary structure" refers to a structure comprising materials having different characteristics joined together to form an integral entity such that the materials are substantially inseparable physically, and the unitary structure exhibits properties resulting from the combination of the materials therein. The materials may be arranged in a face-to-face relationship in the z-dimension, or in a side-by-side relationship in the xy-dimension.

The following detailed description is directed toward absorbent articles. It is, however, likely that the disclosed articles could be modified such that the overall concept discussed herein could likewise be applied to use in other hygiene or health care products, such as bandages, dressings, wipes, bibs, surgical drapes, surgical gowns, and the like.

### ABSORBENT ARTICLE

The absorbent articles of the present invention comprise a topsheet having a garment facing surface and a bodyfacing surface, a backsheet having a garment facing surface and a bodyfacing surface, an absorbent core having two opposing longitudinal edges and two opposing right and left transverse edges and wherein said core is disposed between said body surface of the backsheet and the garment facing surface of the topsheet; and wherein the graphic is disposed on the bodyfacing surface of the backsheet and is partially covered by the core.

In certain embodiments, the absorbent articles may additionally include one or more components selected from the group consisting of an outer cover, side panels, a cuff, an elastic feature, a fastening system, and combinations thereof.

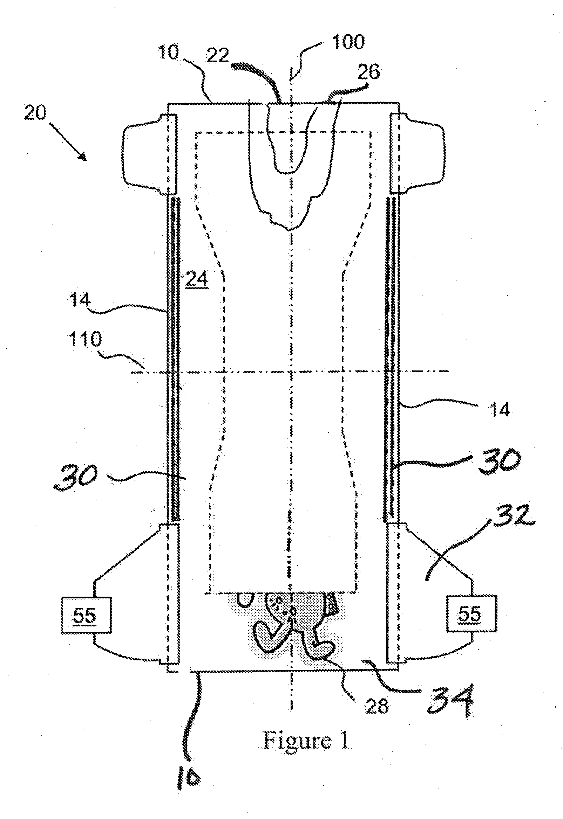

Figure 1 is a top plan view of a diaper of the present invention in a flat-out state with portion of the structure being cut away to more clearly show the construction of the diaper 20. The portion of the diaper 20 which faces the wearer is oriented towards the viewer. This diaper 20 comprises a topsheet 24; a backsheet 22; an absorbent core 26 which is preferably positioned between at least a portion of the topsheet 24 and the backsheet 22; side panels 32; elasticized leg cuffs 30; an elastic waist feature 34; and a fastening system generally designated 55. The periphery of the diaper 20 is defined by the outer edges of the diaper 20 in which opposing right and left longitudinal edges 14 run generally parallel to the longitudinal centerline 100 of the diaper 20 and transverse edges 10 run between the longitudinal edges 14 generally parallel to the lateral centerline 110 of the diaper 20. A graphic 28 is partially hidden by the absorbent core 26 as the graphic is disposed beneath the core in the construction of the diaper 20. In Figure 1, graphic 28 is disposed at the rear of the diaper 20 but in alternative embodiments the graphic may be disposed at the front of the diaper.

In alternative embodiments, the article may be preformed by the manufacturer to create a pant. The term "pant", as used herein, refers to disposable garments having a waist opening and leg openings designed for infant or adult wearers. A pant may be placed in position on the wearer by inserting the wearer's legs into the leg openings and sliding the pant into position about the wearer's lower torso. A pant may be preformed by any suitable technique including, but not limited to, joining together portions of the article using refastenable and/or non-refastenable bonds (e.g., seam, weld, adhesive, cohesive bond, fastener, etc.). While the term "pant" is used herein, pants are also commonly referred to as "closed diapers", "prefastened diapers", "pull-on diapers", "training pants" and "diaper-pants". Suitable pants are disclosed in U.S. Patent Nos. 5246433; 5569234, 6120487, 6120489, 4940464, 5092861, 5897545, 5957908, U.S. Patent Publication 2003/0233082A1, U.S. Patent Nos. 3860003, 4636207, 4695278, 4704115, 4795454, 4900317, 4909803 (Reissued as USRE34920), 5085654, 5492751, 6476288, 6627787, 5507760, 5609587, 5635191, 5643588, 6118041, SIR H1630, 5246433, 5769838, 5899895, 5899896, and 6120487.

The chassis of the diaper 20 comprises the main body of the diaper 20. The chassis comprises at least a portion of the absorbent core 26 and preferably an outer covering including the topsheet 24 and/or the backsheet 22. If the absorbent article comprises a separate holder and a liner, the chassis generally comprises the holder and the liner. (For example, the holder may comprise one or more layers of material to form the outer cover of the article and the liner may comprise an absorbent assembly including a topsheet, a backsheet, and an absorbent core. In such cases, the holder and/or the liner may include a fastening element which is used to hold the liner in place throughout the time of use.) For unitary absorbent articles, the chassis comprises the main structure of the diaper with other features added to form the composite diaper structure. While the topsheet 24, the backsheet 22, and the absorbent core 26 may be assembled in a variety of well known configurations, preferred diaper configurations are described generally in U.S. Pat. No. 3860003; U.S. Pat. No. 5151092; and U.S. Pat. No. 5221274; U.S. Pat. No. 5554145; U.S. Pat. No. 5569234; U.S. Pat. No. 5580411; and U.S. Patent No. 6004306.

### TOPSHEET

The absorbent articles of the present invention comprise a topsheet. The topsheet is preferably compliant, soft feeling, and non-irritating to the wearer's skin. It can be elastically stretchable in one or two directions. Further, the topsheet is liquid pervious, permitting liquids (e.g., menses, urine, and/or runny feces) to readily penetrate through its thickness. A suitable topsheet can be manufactured from a wide range of materials such as woven and nonwoven materials; apertured or hydroformed thermoplastic films; porous foams; reticulated foams; reticulated thermoplastic films; and thermoplastic scrims. Suitable woven and nonwoven materials may comprise of natural fibers such as wood or cotton fibers; synthetic fibers such as polyester, polypropylene, or polyethylene fibers; or combinations thereof. If the topsheet includes fibers, the fibers may be spunbond, carded, wet-laid, meltblown, hydroentangled, or otherwise processed as is known in the art. One suitable topsheet comprising a web of staple-length polypropylene fibers is manufactured by Veratec, Inc., a Division of International Paper Company, of Walpole, MA under the designation P-8.

Preferred topsheet for use in the present invention are selected from high loft nonwoven topsheets and apertured film topsheet. Apertured film topsheet typically are pervious to bodily exudates, yet non-absorbent, and have a reduced tendency to allow fluids to pass back through and rewet the wearer's skin. Suitable apertured films include those described in U. S. Pat. Nos. 5628097, 5916661, 6545197, 6107539, and PCT Patent Publication WO 00/69382 A2.

Further, suitable topsheet materials for depositing solid excretions thereon may include nonwovens having apertures, which are at least in the portions that are aligned with the feces deposition region of the article. Suitable apertured nonwovens are described in more detail in U.S. Patents 6414215, 5342338, and 5941864 and U.S. Patent Publication 2002/017376. In another embodiment of feces handling articles, such topsheets can be combined with feces handling members, e.g., underlying such topsheets, and which are further described in the abovementioned patent documents.

Suitable formed film topsheets are described in U.S. Pat. Nos. 3929135, 4324246, 4342314, 4463045, 5006394. Other suitable topsheets may be made in accordance with U.S. Pat. Nos. 4609518 and 4629643. Such formed films are available from The Procter & Gamble Company of Cincinnati, Ohio as "DRI-WEAVE" and from Tredegar Corporation, based in Richmond, VA, as "CLIFF-T."

In certain embodiments, at least a portion of the topsheet is made of a hydrophobic material or is treated to be hydrophobic in order to isolate the wearer's skin from liquids contained in the absorbent core. If the topsheet is made of a hydrophobic material, preferably at least a portion of the upper surface of the topsheet is treated to be hydrophilic so that liquids will transfer through the topsheet more rapidly. The topsheet can be rendered hydrophilic by treating it with a surfactant or by incorporating a surfactant into the topsheet. Suitable methods for treating the topsheet with a surfactant include spraying the topsheet material with the surfactant and/or immersing the material into the surfactant. A more detailed discussion of such a treatment and hydrophilicity is contained in U.S. Pat. Nos. 4988344, 4988345, and 4950254. A more detailed discussion of some suitable methods for incorporating a surfactant in the topsheet 24 can be found in U.S. Statutory Invention Registration No. H1670. Alternatively, the topsheet may include an apertured web or film which is hydrophobic. This may be accomplished by eliminating the hydrophilizing treatment step from the production process and/or applying a hydrophobic treatment to the topsheet, such as a polytetraflouroethylene compound like SCOTCHGUARD or a hydrophobic lotion composition, as described below. In such embodiments, it is preferred that the apertures be large enough to allow the penetration of aqueous fluids like urine without significant resistance.

Any portion of the topsheet may be coated with a lotion as is known in the art. Examples of suitable lotions include those described in U.S. Pat. Nos. 5607760, 5609587, 5635191, 5643588, and 5968025. The lotion may function alone or in combination with another agent as the hydrophobizing treatment described above. The topsheet may also include or be treated with antibacterial agents, some examples of which are disclosed in PCT Publication No. WO 95/24173. Further, the topsheet, the outer cover or any portion of the topsheet or outer cover may be embossed and/or matte finished to provide a more cloth like appearance.

The topsheet may comprise one or more apertures to ease penetration of exudates therethrough, such as urine and/or feces (solid, semi-solid, or liquid). The size of at least the primary aperture is important in achieving the desired waste encapsulation performance. If the primary aperture is too small, the waste may not pass through the aperture, either due to poor alignment of the waste source and the aperture location or due to fecal masses having a diameter greater than the aperture. If the aperture is too large, the area of skin that may be contaminated by"rewet'(from the article) is increased. Typically, the aperture should have an area of between about 10 cm² and about 50 cm². The aperture preferably has an area of between about 15 cm² and 35 cm².

Further, the topsheet may be fully or partially elasticated or may be foreshortened so as to provide a void space between the topsheet and the core. Exemplary structures including elasticized or foreshortened topsheets are described in more detail in U.S. Pat. Nos. 4892536, 4990147, 5037416, and 5269775.

### BACKSHEET

The backsheet may be impervious to fluids (e.g., menses, urine, and/or runny feces) and may be manufactured from a thin plastic film, although other flexible liquid impervious materials may also be used. As used herein, the term "flexible" refers to materials which are compliant and will readily conform to the general shape and contours of the human body. The backsheet prevents the exudates absorbed and contained in the absorbent core from wetting articles which contact the absorbent article such as bedsheets, pants, pajamas and undergarments. The backsheet may thus comprise a woven or nonwoven material, polymeric films such as thermoplastic films of polyethylene or polypropylene, and/or composite materials such as a film-coated nonwoven material (i.e., having an inner film layer and an outer nonwoven layer). A suitable backsheet is a polyethylene film having a thickness of from about 0.012 mm (0.5 mils) to about 0.051 mm (2.0 mils). Exemplary polyethylene films are manufactured by Clopay Corporation of Cincinnati, Ohio, under the designation BR-120 and BR-121 and by Tredegar Film Products of Terre Haute, Ind., under the designation XP-39385. The backsheet may be embossed and/or matte-finished to provide a more clothlike appearance. Further, the backsheet may permit vapors to escape from the absorbent core (i.e., the backsheet is breathable) while still preventing exudates from passing through the backsheet. The size of the backsheet is dictated by the size of the absorbent core and the exact absorbent article design selected.

The backsheet and the topsheet are positioned adjacent a garment-facing surface and a bodyfacing surface, respectively, of the absorbent core. The absorbent core may be joined with the topsheet, the backsheet, or both in any manner as is known by attachment means such as those well known in the art. However, embodiments of the present invention are envisioned wherein portions of the entire absorbent core are unattached to one or both of the topsheet and the backsheet.

For example, the backsheet and/or the topsheet may be secured to the absorbent core or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minn. under the designation HL-1258, HL-1358, or HL-2031. The attachment means may comprise an open pattern network of filaments of adhesive as is disclosed in US Pat. No. 4573986. An exemplary attachment means of an open pattern network of filaments comprises several lines of adhesive filaments swirled into a spiral pattern such as illustrated by the apparatus and method shown in US Pat. Nos. 3911173, 4785996 and 4842666. Alternatively, the attachment means may comprise heat bonds, pressure bonds, ultrasonic bonds, dynamic mechanical bonds, or any other suitable attachment means or combinations of these attachment means as are known in the art.

The backsheet may include an inner and outer layer, each of which can be bonded to the other by a variety of means known in the art, including thermal bonds, adhesive bonds, ultrasonic lamination, or the like. Intimate bonding of the inner and outer layers in the vicinity of the graphic reduces light diffraction and thus improves the brightness and overall visibility of graphic. Adhesive bonding can also be accomplished using adhesive slot coating, high frequency oscillation patterns, for example in swirl or spray patterns, and other fine denier and/or high coverage application techniques. Suitable laminate adhesives, which can be applied continuously or intermittently, can be obtained from Findley Adhesives, Inc. or from National Starch and Chemical Company.

The outer layer of the backsheet can be made in a variety of forms using different processes. For example, the outer layer may be formed as a carded web, a bonded carded web, a spunbond web, a needled fabric, a woven fabric, or the like to provide a generally cloth-like texture to the wearer. Other additives such as titanium dioxide can represent about 0.5% or less, particularly about 0.3% or less, of the outer layer. In one particular embodiment, the outer layer comprises a spunbond web formed of about 99.5 to 100% polypropylene resin and about 0.5% or less other additives. The outer layer is desirably a lightweight material having a basis weight of about 15 to about 30 gsm and more preferably from about 15 to about 25 gsm.

### ABSORBENT CORE

The articles of the present invention additionally comprise one or more absorbent cores. The absorbent core is at least partially disposed between the topsheet and the backsheet and may take on any size or shape that is compatible with the disposable absorbent article. Exemplary absorbent structures for use as the absorbent core of the present invention that have achieved wide acceptance and commercial success are described in US Pat. Nos. 4610678, 4673402, and 4888231, and 4834735. The absorbent core may further comprise the dual core system containing an acquisition/distribution core of chemically stiffened fibers positioned over an absorbent storage core as detailed in U.S. Pat. Nos. 5234423 and 5147345.

In general, the absorbent core is capable of absorbing or retaining liquids (e.g., menses, urine, and/or other body exudates). The absorbent core is preferably compressible, conformable, and non-irritating to the wearer's skin. The absorbent core may be manufactured in a wide variety of sizes and shapes (e.g., rectangular, oval, hourglass, "T shaped, dog bone, asymmetric, etc.). In general, the absorbent core exhibits a pitch (or length) of from about 50% to about 100% of a pitch exhibited by the backsheet of the article. In other embodiments, the pitch of said core is from about 65% to about 85% of the pitch of said backsheet. Alternatively, the pitch of said core is from about 75% to about 85% of the pitch of said backsheet.

The absorbent core may include any of a wide variety of liquid-absorbent materials commonly used in absorbent articles, such as comminuted wood pulp, which is generally referred to as airfelt. Examples of other suitable absorbent materials for use in the absorbent core include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials; or any equivalent material or combinations of materials, or mixtures of these.

As discussed herein "absorbent gelling materials" and "superabsorbent polymers' are those materials that, upon contact with aqueous fluids, such as bodily fluids, imbibes such fluids and form hydrogels. These absorbent gelling materials are typically capable of absorbing large quantities of aqueous bodily fluids, and further capable of retaining such absorbed fluids under moderate pressures. These absorbent gelling materials are typically in the form of discrete, nonfibrous particles. Other forms, such as fibers, foams, sheets, strips, or other macrostructures, are also suitable for use herein. Suitable absorbent gelling materials in the form of open cell foams may include those disclosed in U.S. Pat. Nos. 3563243, 4554297, 4740520, and 5260345.

The configuration and construction of the absorbent core may also be varied (e.g., the absorbent core may have varying caliper zones and/or have a profile so as to be thicker in the center; hydrophilic gradients; superabsorbent gradients; or lower average density and lower average basis weight zones, e.g., acquisition zones; or may comprise one or more layers or structures). The total absorbent capacity of the absorbent core should, however, be compatible with the design loading and the intended use of the absorbent article. Further, the size and absorbent capacity of the absorbent core may be varied to accommodate different uses such as diapers, incontinence pads, pantiliners, regular sanitary napkins, and overnight sanitary napkins, and to accommodate wearers ranging from infants to adults. The absorbent core can include other absorbent components that are often used in absorbent articles, for example, a dusting layer, a wicking or acquisition layer, or a secondary topsheet for increasing the wearer's comfort.

In certain embodiments of the present invention, the absorbent article may also include a sublayer disposed between the topsheet and the backsheet. The sublayer may be any material or structure capable of accepting, storing or immobilizing bodily exudates. Thus, the sublayer may include a single material or a number of materials operatively associated with each other. Further, the sublayer may be integral with another element of the absorbent article or may be one or more separate elements joined directly or indirectly with one or more elements of the article. Further, the sublayer may include a structure that is separate from the core or may include or be part of at least a portion of the core.

Suitable materials for use as the sublayer may include large cell open foams, macroporous compression resistant nonwoven highlofts, large size particulate forms of open and closed cell foams (macro and/or nicroporous), highloft nonwovens, polyolefin, polystyrene, polyurethane foams or particles, structures comprising a multiplicity of vertically oriented looped strands of fibers, absorbent core structures described above having punched holes or depressions, and the like. One embodiment of a sublayer includes a mechanical fastening loop landing element, having an uncompressed thickness of about 1.5 millimeters available as XPL-7124 from the 3M Corporation of Minneapolis, Minnesota. Another embodiment includes a 6 denier, crimped and resin-bonded nonwoven highloft having a basis weight of 110 grams per square meter and an uncompressed thickness of 7.9 millimeters which is available from the Glit Company of Wrens, Georgia. Other suitable absorbent and nonabsorbent sublayers are described in U.S. Patent Nos. 6680422 and 5941864. Further, the sublayer, or any portion thereof, may include or be coated with a lotion or other known substances to add, enhance or change the performance or other characteristics of the element.

Additionally, suitable absorbent cores may contain reduced amounts of cellulosic airfelt material. For instance, such cores may comprise less than about 40%, 30%, 20%, 10%, 5%, or even 1%. Such a core comprises primarily absorbent gelling material in amounts of at least about 60%, 70%, 80%, 85%, 90%, 95%, or even about 100%, where the remainder of the core comprises a microfiber glue (if applicable). Such cores, microfiber glues, and absorbent gelling materials are described in US Patent Nos. 5599335, 5562646, 5669894, 6790798, US Patent Publication 2004/0158212A1, 2004/0097895A1, US Application Ser. Nos. 10/758,375, and 10/758,138, both filed on January 15, 2004.

In further embodiments, the articles of the present invention may further comprise a wetness sensation member. This member may be disposed in various locations within the article. For instance, the wetness sensation member may be disposed on the topsheet. The member may comprise a permeable layer and an impermeable layer, wherein urine passes through the permeable layer and not through the impermeable layer such that a wearer is made of aware of the fact that urination has occurred as a result of the "wet" feeling. Suitable members are detailed in US Patent 6627786.

### GRAPHIC

The disposable absorbent articles of the present invention further comprise a graphic that is disposed on the bodyfacing surface of the backsheet and that is partially covered by the core. The term "disposed on," and variations thereof are intended to mean that one element can be integral with another element, or that one element can be a separate structure bonded to or placed with or placed near another element. Thus, the graphic can be formed or applied directly or indirectly to the bodyfacing surface of the backsheet. In particular embodiments, the graphic can be printed, or otherwise applied directly on a layer of the backsheet. In certain embodiments, the graphic is partially visible along an edge of the absorbent core, i.e., one or more of the right or left longitudinal edges or the front or back transverse edges. In either instance, the edge of the core may conform to a shape that is selected from the group consisting of a convex curve, a concave curve, a straight line, and combinations thereof. The edge may take on a variety of other shapes as well including irregular shapes.

The graphic may also be disposed on one or more surfaces of the backsheet that are bodyfacing. The first of these surfaces may be the interior surface of the film layer of the backsheet. The second surface may be the exterior surface of the film layer of the backsheet. The third surface of the backsheet may be the interior surface of the nonwoven outer cover of the backsheet. In the latter two instances, it is more likely that the film layer of the backsheet will be at least partially translucent to allow for viewing of the graphic on the underlying nonwoven outer cover.

The graphic may take various forms itself. For instance, the graphic can include object graphics, including but not limited to character graphics, inanimate objects, background graphics, or the like. The term "character graphic" is used herein to refer to a graphic containing an anthropomorphous image, and in particular an image having or suggesting human form or appearance which ascribes human motivations, characteristics or behavior to inanimate objects, animals, natural phenomena, cartoon characters, or the like. Ideally, the character graphic would be suitable for children's underwear and could be utilized to motivate children to wear the absorbent article. To that end, the character graphics can be associated with popular characters in the media, advertising or well known in a particular culture. Typically, they are characters that the wearer or caregiver has some predisposed inclination toward In certain instances, the wearer can imagine himself or herself taking the place of the character. Suitable character graphics can include animals, people, inanimate objects, natural phenomena, cartoon characters, or the like that can or cannot be provided with human features such as arms, legs, facial features or the like.

The graphic is a semi-permanent graphic which is a combination of a permanent graphic, a fading graphic (also known as a disappearing graphic. As used herein permanent graphic' refers to a graphic that is applied to a substrate using one or more non-responsive ink compositions such that the graphic does not dissipate when contacted with water or urine. As used herein 'fading graphic" or "disappearing graphic' refers to a graphic that is applied to a substrate using one or more responsive ink compositions such that the graphic partially or completely dissipates when contacted with water or urine. As used herein "semi-permanent graphic refers to a graphic that is applied to a substrate using a semi-responsive ink composition such that the graphic partially dissipates when contacted with water or urine but leaves a recognizably complete alternate graphic.

According to the present invention, the one or more non-responsive ink compositions comprise from about 1%, 2%, or 4% to about 6%, 8% or 10%, by weight, of solid pigment particles. The solid pigments particles suitable for use in the present invention include, but not limited to examples, pigment Yellow (C.I. 14), pigment Red (C.I. 48:3), pigment Blue (C.I. 15:4), Pigment Black (C.I. 7). In particular, suitable non-responsive ink compositions are commercially available from Sun Chemical under the tradenames Sunfast including colors like Violet, Red, Magenta; Spectra PAC, Sunsperse, Sunbrite series inks.

For the one or more responsive ink compositions, these compositions may comprise from about 1%, 2%, or 3% to about 5%, 7%, 8%, or 10%, by weight, of a water soluble dye. For instance, suitable water soluble inks suitable for these compositions include, but are not limited to, direct dyes, acid dyes, base dyes, and various solvent soluble dyes. Particular dyes that are suitable include FD&C Blue 1 (C.I. 42090:2), D&C Red 6(C.I. 15850), D&C Red 7(C.I. 15850:1), D&C Red 9(C.I. 15585:1), D&C Red 21(C.I. 45380:2), D&C Red 22(C.I. 45380:3), D&C Red 27(C.I. 45410:1), D&C Red 28(C.I. 45410:2), D&C Red 30(C.I. 73360), D&C Red 33(C.I. 17200), D&C Red 34(C.I. 15880:1), and FD&C Yellow 5(C.I. 19140:1), FD&C Yellow 6(C.I. 15985:1), FD&C Yellow 10(C.I. 47005:1), and D&C Orange 5(C.I. 45370:2), and combinations thereof. Such dyes are commercially available from Sun Chemical as well.

The semi-responsive ink compositions comprise a mixture of one or more non responsive ink compositions and one or more responsive ink compositions.

The solvents of the present invention may be selected depending on the type of ink composition. Suitable solvents are selected from the group consisting of aqueous solvents, non-aqueous solvents, and combinations thereof. Suitable non-aqueous solvents may comprise alcohols. The alcohols may be selected from the group consisting of iso-propyl alcohol, n-propyl alcohol, ethanol, methanol, isopropyl acetate, n-propyl acetate, and combinations thereof. Alternatively, suitable aqueous solvents include water, and combinations thereof.

In preferred embodiments, the graphics can be printed (via flexography, gravure, digital printing, etc.). For such printing, it is envisioned that a multiplicity of additional responsive color compositions may be utilized such that multicolor graphics are achieved prior to or upon wetting of the article. Regardless of the type of printing method employed, preferred speeds for printing range from about 50 m/min to about 500 m/min, preferably from about 100 m/min to about 300 m/min. In preferred embodiments, the printing speed is set at about 200 m/min. Additionally a multicolor print press is utilized to print the graphic on the backsheet material with the color composition and varnish coating being printed in succession from different print stations.

Additionally, other graphics may be sprayed or otherwise applied directly on a layer of the backsheet. For instance, in other embodiments, the graphics can be applied to a layer placed with or near the backsheet, such as a substrate associated with the absorbent assembly, including but not limited to tissue layers, liquid handling layers, absorbent layers, or the like.

The graphics for use with the present invention can be located on or against either surface (i.e., garment-facing (aka exterior) surface or body-facing (aka interior) surface) of the backsheet provided the graphics remain visible from the exterior of the product when wetted. In preferred embodiments, the wetness indicator graphic is printed on the interior surface of the backsheet.

In a preferred embodiments, the absorbent article further comprises at least a first cuff for providing improved containment of liquids and other body exudates; an elastic waist feature that provides improved fit and containment; and a fastening system which forms a side closure which maintains the first waist region and the second waist region in an overlapping configuration such that lateral tensions are maintained around the circumference of the absorbent article to maintain the absorbent article on the wearer. The absorbent article may also comprise elasticized side panels (not shown) in the waist regions and to provide an elastically extensible feature that provides a more comfortable and contouring fit and more effective application of the absorbent article.

The "elasticized' leg cuffs can be constructed in a number of different configurations, including those described in US Pat. Nos. 3860003, 4636207, 4695278, 4704115, 4795454, 4900317, 4909803 (Reissued as USRE34920), 5085654, 5492751, 6476288 and SIR H1630.

For example, the absorbent article may include one ore more first cuffs that provide improved containment of liquids and other body exudates. First cuffs may also be referred to as outer leg cuff, leg bands, side flaps, leg cuffs or elastic cuffs. U.S. Pat. No. 3860003 describes a disposable diaper which provides a contractible leg opening having a side flap and one or more elastic members to provide an elasticized leg cuff.

Additionally, an absorbent article of the present invention may include one or more second cuffs that also provide improved containment of liquids and other body exudates. Second cuffs may also be referred to as barrier leg cuffs, inner leg cuffs or "stand-up" elasticized flaps. U.S. Pat. Nos. 4808178 and 4909803 (Reissued as USRE34920) describe disposable diapers having "stand-up" elasticized flaps that improve the containment of the leg regions.

First cuff and second cuff may both be provided by way of a dual cuff, as exampled in U.S. Pat. Nos. 4695278 and 4795454. Additional cuffs may be provided in an article of the present invention as detailed in US Statutory Invention Registration H1630.

Another component that may be included in the articles or composites of the present invention is a hydrophobic surface coating as disclosed in copending US Patent Application Serial No. 11/055743 (P&G Case 9528M), which was filed on February 10, 2005. This hydrophobic surface coating may be paired with the barrier coating of the present invention on the barrier layer or may be disposed adjacent to one or more additional components of the absorbent articles or composites of the present invention. For instance, this hydrophobic surface coating may be disposed on an interior surface of one or more leg cuffs, waist portions, or other areas of the absorbent article.

Moreover, Applicants have found that the present invention is also directed to a method of incorporating a partially visible graphic into an absorbent article. This method comprises the steps of disposing a graphic onto a bodyfacing surface of a backsheet of an absorbent article and partially covering said graphic with an absorbent core. The various embodiments discussed earlier may also be employed with respect to this method. Likewise, once this method is employed to provide such absorbent articles, these articles may be packaged in a kit suitable for purchase by caregivers. An absorbent article kit according to the present invention comprises one or more absorbent articles described herein and an overwrap containing said one or more articles wherein a garment facing surface of said articles and said graphic is visible through said overwrap. The overwrap may be a nonwoven web or film. In the case of a nonwoven web, it may comprise natural fibers, synthetic fibers, and combinations thereof. Suitable fibers include those detailed in reference to the topsheet discussed earlier. The film may comprise one or more polyolefins.

### EXAMPLES (both outside the scope of the claimed invention)

### Example 1

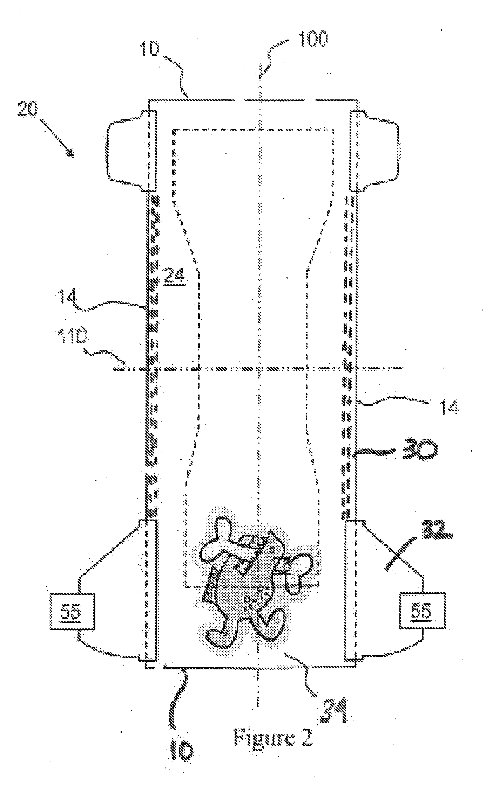

An absorbent article which is a diaper, training pant, or adult incontinence product is made as detailed in any one of US Patents 3860003, 4636207, 4695278, 4704115, 4795454, 49170317, 4909803 (Reissued as USRE34920), 5085654, 5492751, 6476288, 6627787, 5507760, 5609587, 5635191, 5643588, 6118041, SIR H1630, 5246433, 5769838, 5899895, 5899896, and 6120487. The backsheet of the disclosed absorbent articles are printed with a character graphic of an anthropomorphous character (e.g., a cartoon dog) that is facing the core. The character graphic is a semi-permanent graphic and is printed about I" bellow the cut from the back. The graphic is printed using non-responsive ink compositions, particularly pigment Blue (C.I. 15:4) and pigment Black (C.I. 7). The graphic dog character has an eye about 2' below the cut line from the back as shown in Figure 1 where the bodyfacing surface of a topsheet of the article is viewable. The core is located about 2.2' below the cut line from the back. One or both of the eye(s) of the dog is located just above the edge of the absorbent core. The eye(s) can be seen through the topsheet while the diaper was opened and ready to be placed under the baby. Figure 2 depicts a garment facing surface of the backsheet of the article of Figure 1.

### Example 2

An absorbent article as described in Example 1 wherein a responsive ink composition was also included in the character graphic to color the bone. In effect, the graphic is a semi-permanent graphic. This responsive ink composition comprises a water soluble dye selected from the group consisting of FD&C Yellow 5, FD&C Yellow 6, FD&C Yellow 10, D&C Orange 5, and combinations thereof. This ink was used to color the bone such that the color dissipates when contacted with urine. This may also serve as an indicator to the caregiver that the diaper should be removed.

## Claims

1. An absorbent article (20) comprising:
a) a backsheet (22) having a garment facing surface and a bodyfacing surface;
b) a topsheet (24) having a garment facing surface and a bodyfacing surface;
c) an absorbent core (26) having two opposing longitudinal edges (14) and two opposing transverse edges (10) and wherein said core is disposed between said bodyfacing surface of said backsheet and said garment facing surface of said topsheet; and wherein a graphic (28) is disposed on said bodyfacing surface of said backsheet and is partially covered by said core.
**characterized in that** said graphic is a semi-permanent graphic and comprises a semi-responsive ink composition,
the semi-responsive ink composition comprising a mixture of one or more non-responsive ink compositions and one or more responsive ink compositions,
wherein the one or more non-responsive ink compositions comprise from 1% to 10% by weight of solid pigment particles and
the one or more responsive ink compositions comprise from 1% to 10% by weight of a water soluble dye.

2. The absorbent article of claim 1 wherein said absorbent core exhibits a pitch of from 50% to 100% of a pitch exhibited by the backsheet of the article.

3. The absorbent article of any preceding claim wherein said article further comprises at least two leg cuffs (30) that run along said article on at least a right or left longitudinal edge.

4. The absorbent articles of any preceding claim wherein at least the transverse edge that is adjacent said partially exposed graphic conforms to a shape selected from the group consisting of a convex curve, a concave curve, a straight line, an irregular shape, and combinations thereof.

5. The absorbent article of any preceding claim wherein said article further comprises functional elements selected from the group consisting of mechanical fasteners, elastic side panels, adhesive fasteners, waistbands, landing zones, and combinations thereof.

6. The absorbent article of any preceding claim wherein said backsheet feather comprises a film and a nonwoven outercover.

7. A method of incorporating a partially visible graphic (28) intro an absorbent article (20), said method comprising the steps of:
a. disposing a graphic onto a bodyfacing surface of a backsheet (22) of an absorbent article; and
b. partially covering said graphic with an absorbent core (26).
**characterized in that** said graphic is a semi-permanent graphic and comprises a semi-responsive ink composition, the semi-responsive ink composition comprising a mixture of one or more non-responsive ink compositions and one or more responsive ink compositions,
wherein the one or more non-responsive ink compositions comprise from 1% to 10% by weight of solid pigment particles and
the one or more responsive ink compositions comprise from 1% to 10% by weight of a water soluble dye.

## Patentansprüche

1. Absorptionsartikel (20), umfassend:
a) eine Unterschicht (22) mit einer kleidungsseitigen Oberfläche und einer körperseitigen Oberfläche;
b) eine Oberschicht (24) mit einer kleidungsseitigen Oberfläche und einer körperseitigen Oberfläche;
c) einen Absorptionskern (26) mit zwei gegenüberliegenden Längsrändern (14) und zwei gegenüberliegenden Querrändern (10) und wobei der Kern zwischen der körperseitigen Oberfläche der Unterschicht und der kleidungsseitigen Oberfläche der Oberschicht angeordnet ist; und wobei eine Grafik (28) auf der körperseitigen Oberfläche der Unterschicht angeordnet ist und teilweise von dem Kern bedeckt wird.
**dadurch gekennzeichnet, dass** die Grafik eine halbpermanente Grafik ist und eine halbreagierende Tintenzusammensetzung umfasst, wobei die halbreagierende Tintenzusammensetzung eine Mischung aus einer oder mehreren nichtreagierenden Tintenzusammensetzungen und einer oder mehreren reagierenden Tintenzusammensetzungen umfasst, wobei die eine oder mehreren nichtreagierenden Tintenzusammensetzungen von 1 Gew.-% bis 10 Gew.-% feste Pigmentteilchen umfassen und die eine oder mehreren reagierenden Tintenzusammensetzungen von 1 Gew.-% bis 10 Gew.-% einen wasserlöslichen Farbstoff umfassen.

2. Absorptionsartikel nach Anspruch 1, wobei der Absorptionskern eine Länge von ungefähr 50 % bis ungefähr 100 % einer Länge aufweist, die die Unterschicht des Artikels aufweist.

3. Absorptionsartikel nach einem der vorstehenden Ansprüche, wobei der Artikel ferner mindestens zwei Beinbündchen (30) umfasst, die entlang des Artikels an mindestens einem rechten oder linken Längsrand verlaufen.

4. Absorptionsartikel nach einem der vorstehenden Ansprüche, wobei mindestens der Querrand, der an die teilweise freiliegende Grafik angrenzt, einer Form entspricht, die ausgewählt ist aus der Gruppe, bestehend aus einer konvexen Kurve, einer konkaven Kurve, einer geraden Linie, einer unregelmäßigen Form und Kombinationen davon.

5. Absorptionsartikel nach einem der vorstehenden Ansprüche, wobei der Artikel ferner funktionelle Elemente umfasst, die ausgewählt sind aus der Gruppe, bestehend aus mechanischen Befestigungsmitteln, elastischen Seitenfeldern, Haft-Befestigungsmitteln, Taillenbändern, Anlegebereichen und Kombinationen davon.

6. Absorptionsartikel nach einem der vorstehenden Ansprüche, wobei die Unterschicht ferner eine Folie und ein Vliesstoffdeckblatt umfasst.

7. Verfahren zum Einbeziehen einer teilweise sichtbaren Grafik (28) in einen Absorptionsartikel (20), wobei das Verfahren die folgenden Schritte umfasst:
a. Anordnen einer Grafik auf einer körperseitigen Oberfläche einer Unterschicht (22) eines Absorptionsartikels; und
b. teilweise Abdecken der Grafik mit einem Absorptionskern (26).
**dadurch gekennzeichnet, dass** die Grafik eine halbpermanente Grafik ist und eine halbreagierende Tintenzusammensetzung umfasst, wobei die halbreagierende Tintenzusammensetzung eine Mischung aus einer oder mehreren nichtreagierenden Tintenzusammensetzungen und einer oder mehreren reagierenden Tintenzusammensetzungen umfasst, wobei die eine oder mehreren nichtreagierenden Tintenzusammensetzungen von 1 Gew.-% bis 10 Gew.-% feste Pigmentteilchen umfassen und die eine oder mehreren reagierenden Tintenzusammensetzungen von 1 Gew.-% bis 10 Gew.-% einen wasserlöslichen Farbstoff umfassen.

## Revendications

1. Article absorbant (20) comprenant:
a) une feuille de fond (22) ayant une surface faisant face au vêtement et une surface faisant face au corps ;
b) une feuille de dessus (24) ayant une surface faisant face au vêtement et une surface faisant face au corps ;
c) une âme absorbante (26) ayant deux bords longitudinaux opposés (14) et deux bords transversaux opposés (10) et dans lequel ladite âme est disposée entre ladite surface faisant face au corps de ladite feuille de fond et ladite surface faisant face au vêtement de ladite feuille de dessus ; et dans lequel un motif (28) est disposé sur ladite surface faisant face au corps de ladite feuille de fond et est partiellement couvert par ladite âme.
**caractérisé en ce que** ledit motif est un motif semi-permanent, et comprend une composition d'encre semi-réactive, la composition d'encre semi-réactive comprenant un mélange d'une ou plusieurs compositions d'encre non-réactive et une ou plusieurs compositions d'encre réactive, où l'une ou les plusieurs compositions d'encre non réactive comprennent de 1 % à 10 % en poids de particules de pigment solides et l'une ou les plusieurs compositions d'encre réactive comprennent de 1 % à 10 % en poids d'une teinture hydrosoluble.

2. Article absorbant selon la revendication 1, où ladite âme absorbante présente un espacement allant de 50 % à 100 % d'un espacement présenté par la feuille de fond de l'article.

3. Article absorbant selon l'une quelconque des revendications précédentes, où ledit article comprend en outre au moins deux revers de jambe (30) qui s'étendent le long dudit article sur au moins un bord longitudinal droit ou gauche.

4. Article absorbant selon l'une quelconque des revendications précédentes, dans lequel au moins le bord transversal qui est adjacent audit motif partiellement exposé s'adapte à une forme choisie dans le groupe constitué d'une courbe convexe, une courbe concave, une ligne droite, une forme irrégulière, et leurs combinaisons.

5. Article absorbant selon l'une quelconque des revendications précédentes, où ledit article comprend, en outre, des éléments fonctionnels choisis dans le groupe constitué de fermoirs mécaniques, pans latéraux élastiques, fermoirs adhésifs, ceintures, zones de réception, et leurs combinaisons.

6. Article absorbant selon l'une quelconque des revendications précédentes, dans lequel ladite feuille de fond comprend, en outre, un film et une enveloppe extérieure non tissée.

7. Procédé d'incorporation d'un motif partiellement visible (28) dans un article absorbant (20), ledit procédé comprenant les étapes consistant à :
a. disposer un motif sur une surface faisant face au corps d'une feuille de fond (22) d'un article absorbant ; et
b. couvrir partiellement ledit motif avec une âme absorbante (26).
**caractérisé en ce que** ledit motif est un motif semi-permanent, et comprend une composition d'encre semi-réactive, la composition d'encre semi-réactive comprenant un mélange d'une ou plusieurs compositions d'encre non-réactive et une ou plusieurs compositions d'encre réactive, où l'une ou les plusieurs compositions d'encre non réactive comprennent de 1 % à 10 % en poids de particules de pigment solides et l'une ou les plusieurs compositions d'encre réactive comprennent de 1 % à 10 % en poids d'une teinture hydrosoluble.
